# EUROPEAN PATENT APPLICATION

(11) **EP 2 113 222 A1**
(43) Date of publication of application: **04.11.2009**
(21) Application number: 08845308.9
(22) Date of filing: 28.10.2008
(51) Int. Cl.: A61C 9/00, A61K 6/10

(54) **FILM FOR DENTAL IMPRESSION AND METHOD OF PRODUCING FILM FOR DENTAL IMPRESSION**

(30) Priority: 31.10.2007 JP 2007284405; 30.11.2007 JP 2007311652; 13.08.2008 WO PCT/JP2008/065053
(71) Applicant: Fukuhara, Yasuhiro, Nara 636-0303 (JP)
(72) Inventor: Fukuhara, Yasuhiro, Nara 636-0303 (JP)
(74) Representative: Schildberg, Peter
(86) International application number: PCT/JP2008/069547
(87) International publication number: WO 2009/057591

(57) **Abstract**

Attention has been focused on combining two types of dental impression materials of different qualities which has not been considered in conventional combined impression, and a dental impression film and a method of manufacturing the dental impression film are provided for making the two parts joinable. The dental impression film is formed of a polyethylene film 10 and polyethylene/polyester films 20. The polyethylene film 10 has, laminated by thermal adhesion on opposite surfaces thereof, respectively, the polyethylene/polyester films 20 having bonding capability between a silicone impression material and an alginate impression material. The dental impression film having bonding capability is interposed between the silicone impression material and alginate impression material. This enables joining of the silicone impression material and alginate impression material, no longer requires the timing of hardening of the impression materials to be matched, and allows a precise impression at low cost using a small amount of silicone impression material.

## Description

### [TECHNICAL FIELD]

This invention relates to a dental impression film for use in combined impression which combines two types of dental impression materials of different qualities, and to a method of manufacturing the dental impression film.

### [BACKGROUND ART]

Conventionally, when producing a crown restoration, a method has generally been carried out to take an impression using an elastic impression material. Known as usual impression materials are an impression material having soluble salt of alginate as a main ingredient (alginate impression material: see Patent Document 1, for example) and a precision impression material having silicone rubber as a main ingredient (silicone impression material).

Comparing the main characteristics of these impression materials, it can be said that a more precise impression can be taken when the silicone impression material is used than when the alginate impression material is used. However, the silicone impression material is several or more times as expensive as the alginate impression material. Thus, when economic efficiency is taken into consideration, it is difficult to use the silicone impression material in a large amount. Therefore, a cost reduction can be achieved by using the silicone impression material in the least possible amount.

On the other hand, as combined impression which combines two types of dental impression materials, an agar/alginate combined impression, for example, is well known. Its characteristic is that both contain a large quantity of water. This poses a problem that a delay in injecting a mold material after removing an impression will result in deformation of the impression due to contraction accompanying evaporation of the water. Further, a good bonding between agar and alginate is an important point. Since the method combines the two types of unhardened materials, it is necessary to press the two materials together while they have sufficient fluidity.
[Patent Document 1]
   Unexamined Patent Publication No. 2008-222672

### [DISCLOSURE OF THE INVENTION]

### [PROBLEM TO BE SOLVED BY THE INVENTION]

However, where two types of dental impression materials are used and an impression is taken from a large area, it can be said that it is very difficult to cause the respective impression materials to harden in exact timing. That is, when alginate is placed over agar after the agar adhering to teeth hardens, the agar and alginate will not join together but the two will separate from each other. Considering that agar loses fluidity and hardens in about 20 seconds after adhering to teeth, it is not easy to match the timing of hardening.

The object of this invention is to provide a dental impression film and a method of manufacturing the dental impression film, which has been made by focusing attention on combining two types of dental impression materials of different qualities, which has not been considered in conventional combined impression, making the two types of dental impression materials of different qualities joinable at a time of impression taking, and which no longer require the timing of hardening of the respective impression materials to be matched, and can take precise impressions at low cost.

### [MEANS FOR SOLVING THE PROBLEM]

The dental impression film according to this invention is a dental impression film for use in a combined impression which overlaps two types of dental impression materials different in quality, wherein the two types of dental impression materials different in quality are made joinable by laminating a layer having bonding capability, or attaching projections having bonding capability, between the dental impression materials having hardened, on opposite surfaces of the dental impression film at a time of impression taking.

This no longer requires the timing of hardening of the impression materials to be matched, and allows a precise impression at low cost.

The dental impression materials may comprise a combination of a silicone impression material and an alginate impression material.

The dental impression film may comprise films of polyethylene and polyester having bonding capability between silicone impression material and alginate impression material, laminated by thermal adhesion on opposite surfaces of a polyethylene film.

The dental impression film may comprise a layer of silicone impression material fixed to one surface of a film having bonding capability between silicone impression material and alginate impression material.

The dental impression film may comprise looped projections, which will become rigidly and inseparably entwined when permeated by and hardened with the alginate impression material, attached to a surface, to be bonded to the alginate impression material, of the film having bonding capability with respect to the silicone impression material.

The method of manufacturing a dental impression film, according to this invention, comprises laying nonwoven resin fabrics on opposite surfaces of a resin film, respectively, to form three layers, laying a virgin pulp under a lowermost nonwoven resin fabric, then winding these around a roll to form a rolled whole, and subsequently heating at a predetermined temperature with a heating device, thereby causing the nonwoven resin fabrics to thermally adhere to the opposite surfaces of the resin film.

The resin film may be a polyethylene film, and the nonwoven resin fabrics may be a double structure fiber of polyethylene/polyester.

The heating temperature by the heating device may be set to or above a heatproof temperature of the resin film and within a range of 118 to 135°C. The heating device may be a high-pressure steam sterilizer.

The dental impression film for use in a combined impression for joining a dental impression material to a solid, according to this invention, has a permeation surface to which the dental impression material is applied and an attachment surface for attaching to the solid, the permeation surface having, laminated thereon a layer having bonding capability, or attached thereto, projections having bonding capability with respect to the dental impression material having hardened, the attachment surface being coated with an adhesive material to be attachable to the solid, thereby being capable of joining the dental impression material to the solid.

According to the dental impression film of this invention, as described above, two types of dental impression materials different in quality are combined and made joinable, which has not been considered in the conventional combined impression, by laminating a layer having bonding capability, or attaching projections having bonding capability, between the dental impression materials having hardened at a time of impression taking, on opposite surfaces of the dental impression film. This enables a precise impression to be taken at low cost using the characteristic of each dental impression material. By interposing the dental impression film, there is no need to match timing of hardening of the dental impression materials. Thus, it becomes easy to carry out a series of operations from malaxation to completion of the impression even when the impression is taken from a large area.

According to the method of manufacturing the dental impression film of this invention, the nonwoven resin fabrics, while retaining their three-dimensional fiber structure, can be made to thermally adhere to the opposite surfaces of the resin film simply and reliably. This facilitates manufacture of goods at a time of mass production, and can supply goods of stable quality at low cost.

### [BRIEF DESCRIPTION OF THE DRAWINGS]

Fig. 1 is an explanatory view showing Embodiment 1 of dental impression film according to this invention;
Fig. 2 is an explanatory view showing Embodiment 2 of dental impression film according to this invention;
Fig. 3 is an explanatory view showing Embodiment 3 of dental impression film according to this invention;
Fig. 4 is an explanatory view showing Embodiment 4 of dental impression film according to this invention;
Fig. 5 is an explanatory view showing Embodiment 5 of dental impression film according to this invention;
Fig. 6 is an explanatory view showing Embodiment 6 of dental impression film according to this invention;
Fig. 7 is an explanatory view showing Embodiment 6 of dental impression film according to this invention;
Fig. 8 is an explanatory view showing Embodiment 6 of dental impression film according to this invention;
Fig. 9 is an explanatory view showing Embodiment 6 of dental impression film according to this invention;
Fig. 10 is an explanatory view showing Embodiment 6 of dental impression film according to this invention;
Fig. 11 is an explanatory view showing Embodiment 7 of dental impression film according to this invention;
Fig. 12 is an explanatory view showing Embodiment 7 of dental impression film according to this invention; and
Fig. 13 is an explanatory view showing Embodiment 7 of dental impression film according to this invention.

### [Description of References]

10 polyethylene film
13 miracle fit (dental impression film)
16 silicone (dental impression material)
20 polyethylene/polyester films
26 alginate (dental impression material)
31 original plate denture (solid)

### [BEST MODE FOR CARRYING OUT THE INVENTION]

One configuration of dental impression film according to this invention will be described hereinafter with reference to the drawings.

The "two types of dental impression materials of different qualities" of this invention are not limited to a combination of a silicone impression material and an alginate impression material, but any combination of dental impression materials conforming to the purport of this invention can be selected. For example, a rubber impression material, an agar impression material and so on may be employed.

### [Embodiment 1]

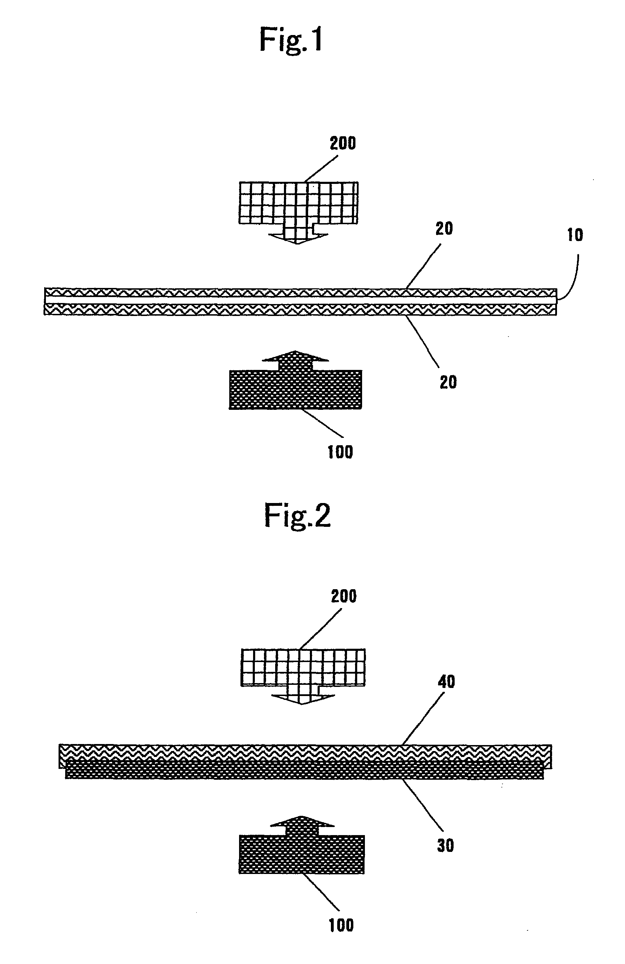

Fig. 1 is an explanatory view showing Embodiment 1 of dental impression film according to this invention.

The dental impression film in this embodiment is formed of a polyethylene film 10 and polyethylene/polyester films 20. As shown in Fig. 1, the polyethylene film 10 has, laminated by thermal adhesion on opposite surfaces thereof, respectively, the polyethylene/polyester films 20 having bonding capability between a silicone impression material and an alginate impression material. This forms a laminated object having three layers.

Here, the quality of material of the surfaces forming the laminated object of dental impression film is not limited to the above. It is possible to employ materials having bonding capability between the silicone impression material and alginate impression material, respectively, and having a structure and thickness not easily separable when hardened.

The structure not easily separable in the laminated object of dental impression film is, for example, a structure having voids permeable by the silicone impression material and alginate impression material, and a structure having entwined fibers. The thickness not easily separable is, for example, such that one layer of polyethylene/polyester film 20 desirably is about 0.1mm - 0.4mm, and more desirably about 0.15mm - 0.25mm. Most desirably, a thickness of 0.2mm can be employed. The thickness of polyethylene film 10 is in the order of 10µm. Thus, the thickness of dental impression film according to Embodiment 1 is substantially determined by the thickness of polyethylene/polyester films 20.

When an impression is taken by a combined impression combining a silicone impression material 100 and an alginate impression material 200 using the dental impression film of this embodiment, the dental impression film having bonding capability is interposed between the dental impression materials, i.e. the silicone impression material 100 and alginate impression material 200 (see Fig. 1). This enables joining of the silicone impression material 100 and alginate impression material 200, which has been impossible heretofore, and allows a precise impression at low cost using a small amount of silicone impression material 100. After the silicone impression material 100 hardens in the state of adhering to one surface of the dental impression film, the alginate impression material 200 can be applied to the other surface of the dental impression film. Since the silicone impression material 100 and alginate impression material 200 can be hardened separately and independently, there is no need to match the timing of hardening of the silicone impression material 100 and alginate impression material 200 when forming an impression. It becomes easy to carry out a series of operations from malaxation to completion of the impression.

The polyethylene film 10 sandwiched between the polyethylene/polyester films 20 in the dental impression film has a function to prohibit permeation of the impression materials, and prevents permeation of the impression materials between the front surface and back surface of the dental impression film. For example, even if the silicone impression material 100 is first applied to one surface of the dental impression film, the silicone impression material 100 is obstructed by the polyethylene film 10, and cannot reach the other surface of the dental impression film. When, in this state, the alginate impression material 200 is applied to the other surface of the dental impression film, the alginate impression material 200 can easily permeate the dental impression film since the polyethylene film 10 has voids permeable by the alginate impression material 200.

### [Embodiment 2]

Fig. 2 is an explanatory view showing Embodiment 2 of dental impression film according to this invention.

The dental impression film in this embodiment is formed of a silicone impression material layer 30 and an adhesive film 40. As shown in Fig. 2, the silicone impression material layer 30 is fixed beforehand to one surface of the adhesive film 40 having bonding capability between silicone impression material and alginate impression material.

Here, the quality of the material employed to form the adhesive film 40 of the dental impression film has bonding capability between silicone impression material and alginate impression material, respectively, and has a quality and thickness not easily separable when hardened.

With such construction, a silicone impression material 100 is applied to one surface of the adhesive film 40 to which the silicone impression material 30 has been fixed. The silicone impression material 100 hardens in the state of being joined to the silicone impression material 30, and sticks to the dental impression film reliably. On the other hand, the adhesive film 40 has a function to bond an alginate impression material 200. Thus, the silicone impression material 100 and alginate impression material 200 are reliably joined through the dental impression film according to Embodiment 2.

That is, when an impression is taken by a combined impression combining the silicone impression material 100 and alginate impression material 200 using the dental impression film of this embodiment, the silicone impression material 100, when hardened, rigidly joins the silicone impression material layer 30, the alginate impression material 200, when hardened, adheres to the adhesive film 40, and the above dental impression film is interposed therebetween (see Fig. 2). As in Embodiment 1, this enables joining of the silicone impression material 100 and alginate impression material 200, which has been impossible heretofore, and allows a precise impression at low cost using a small amount of silicone impression materials 100. As in Embodiment 1, after the silicone impression material 100 hardens in the state of adhering to one surface of the dental impression film, the alginate impression material 200 can be applied to the other surface of the dental impression film. Since the silicone impression material 100 and alginate impression material 200 can be hardened separately and independently, there is no need to match the timing of hardening of the silicone impression material 100 and alginate impression material 200 when forming an impression. It becomes easy to carry out a series of operations from malaxation to completion of the impression.

### [Embodiment 3]

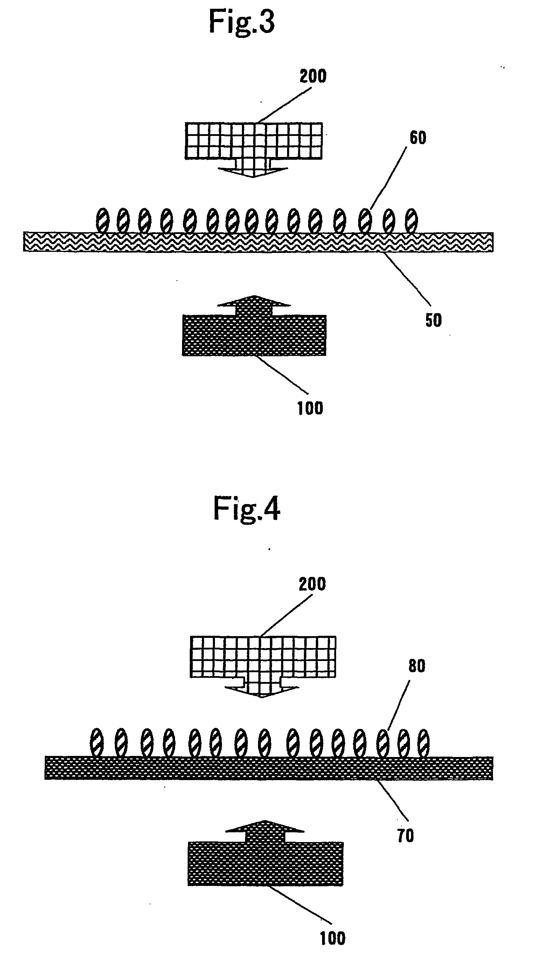

Fig. 3 is an explanatory view showing Embodiment 3 of dental impression film according to this invention.

The dental impression film in this embodiment is formed of an adhesive film 50 and looped projections 60. As shown in Fig. 3, the adhesive film 50 having bonding capability with respect to a silicone impression material has, attached to the surface thereof to be bonded to an alginate impression material, numerous looped projections 60 which will become rigidly and inseparably entwined when permeated by and hardened with the alginate impression material. The surface of the adhesive film 50 to which the looped projections 60 are attached will be called the mounting surface, and the other surface of the adhesive film 50 the surface to be treated.

Here, the quality of the material employed to form the adhesive film 50 of the dental impression film has bonding capability with respect to the silicone impression material, and has a quality and thickness not easily separable when hardened.

When an impression is taken by a combined impression combining a silicone impression material 100 and an alginate impression material 200 using the dental impression film of this embodiment, as shown in Fig. 3, the alginate impression material 200 is applied to the mounting surface of the adhesive film 50, and the silicone impression material 100 to the surface to be treated of the adhesive film 50. The silicone impression material 100, when hardening, permeates the surface to be treated, and adheres to the adhesive film 50. The alginate impression material 200, when hardening, becomes entangled inseparably with the numerous looped projections 60 on the mounting surface. By interposing the above dental impression film (see Fig. 3), as in Embodiment 1 and Embodiment 2, after the silicone impression material 100 hardens in the state of adhering to one surface of the dental impression film, the alginate impression material 200 can be applied to the other surface of the dental impression film. Since the silicone impression material 100 and alginate impression material 200 can be hardened separately and independently, the silicone impression material 100 and alginate impression material 200 can be bonded at a time of forming an impression, which has been impossible heretofore, and which allows a precise impression to be taken at low cost using a small amount of silicone impression materials 100. As in Embodiment 1 and Embodiment 2, there is no need to match the timing of hardening of the silicone impression material 100 and alginate impression material 200. It becomes easy to carry out a series of operations from malaxation to completion of the impression.

### [Embodiment 4]

Fig. 4 is an explanatory view showing Embodiment 4 of dental impression film according to this invention.

The dental impression film in this embodiment is formed of a silicone impression material film 70 and looped projections 80. As shown in Fig. 4, the silicone impression material film 70 has, attached to the surface thereof to be bonded to an alginate impression material, numerous looped projections 80 which will become rigidly and inseparably entwined when permeated by and hardened with the alginate impression material. The surface of the silicone impression material film 70 to which the looped projections 60 are attached will be called the mounting surface, and the other surface of the silicone impression material film 70 the surface to be treated.

When an impression is taken by a combined impression combining a silicone impression material 100 and an alginate impression material 200 using the dental impression film of this embodiment, as shown in Fig. 4, the alginate impression material 200 is applied to the mounting surface of the silicone impression material film 70, and the silicone impression material 100 to the surface to be treated of the silicone impression material film 70. The silicone impression material 100, when hardened, rigidly joins the silicone impression material film 70, the alginate impression material 200, when hardened. The alginate impression material 200, when hardened, becomes entangled inseparably with the numerous looped projections 80 on the mounting surface. By interposing the above dental impression film (see Fig. 4), as in Embodiment 3, the silicone impression material 100 and alginate impression material 200 can be bonded, which has been impossible heretofore, and which allows a precise impression to be taken at low cost using a small amount of silicone impression materials 100. As in Embodiment 3, after the silicone impression material 100 hardens in the state of adhering to one surface of the dental impression film, the alginate impression material 200 can be applied to the other surface of the dental impression film. Since the silicone impression material 100 and alginate impression material 200 can be hardned separately and independently, there is no need to match the timing of hardening of the silicone impression material 100 and alginate impression material 200 when forming an impression. It becomes easy to carry out a series of operations from malaxation to completion of the impression.

### [Embodiment 5]

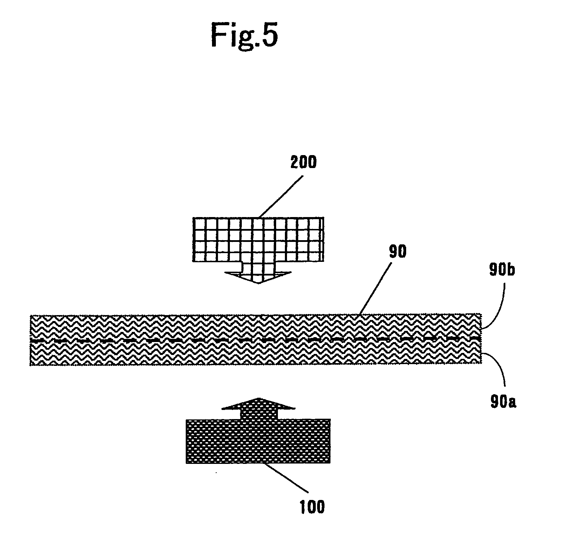

Fig. 5 is an explanatory view showing Embodiment 5 of dental impression film according to this invention.

The dental impression film in this embodiment is formed of an adhesive film 90 having bonding capability between silicone impression material and alginate impression material, respectively, and having a quality and thickness not easily separable when hardened.

When an impression is taken by a combined impression combining the silicone impression material 100 and alginate impression material 200 using the dental impression film of this embodiment, the silicone impression material 100, when hardened, adheres to a lower half region of the adhesive film 90, the alginate impression material 200, when hardened, adheres to an upper half region of the adhesive film 90. By interposing the above dental impression film (see Fig. 5), as in Embodiment 1 and others, after the silicone impression material 100 hardens in the state of adhering to one surface of the dental impression film, the alginate impression material 200 can be applied to the other surface of the dental impression film. Since the silicone impression material 100 and alginate impression material 200 can be hardened separately and independently, the silicone impression material 100 and alginate impression material 200 can be bonded at a time of forming an impression, which has been impossible heretofore, and which allows a precise impression to be taken at low cost using a small amount of silicone impression materials 100. As in Embodiment 1 and others, there is no need to match the timing of hardening of the silicone impression material 100 and alginate impression material 200. It becomes easy to carry out a series of operations from malaxation to completion of the impression.

There are types of silicone impression materials, such as addition type silicone and condensed type silicone. Where bonding with the dental impression film is based on the chemical property of material, only impression materials of the same type (added type or condensed type) can be bonded. Thus, it is necessary to prepare chemical bonding layers with varied qualities of the dental impression film (Embodiment 2 or Embodiment 4 described above) according to the types, and to select an impression material (silicone or the like) at a time of actual impression according to the chemical bonding property. With the dental impression film not relying on chemical bonding (Embodiment 1, Embodiment 3 or Embodiment 5 described above), the impression material is not limited by bonding property.

A method of manufacturing the dental impression film according to this invention will be described hereinafter.

The method of manufacturing the dental impression film according to this invention is a method of manufacturing the dental impression film in which nonwoven resin fabrics are laid on opposite surfaces of a resin film, respectively, to form three layers, a virgin pulp is laid under the lowermost nonwoven resin fabric, then these are wound around a roll to form a rolled whole, and subsequently they are heated at a predetermined temperature with a heating device, thereby causing the nonwoven resin fabrics to thermally adhere to the opposite surfaces of the resin film.

The method of manufacturing the dental impression film according to this invention is not limited to the following embodiments, but the specific contents of constituent elements can be varied as appropriate within a range corresponding to the purport of this invention.

### (Manufacturing Process 1)

First, nonwoven resin fabrics are laid on opposite surfaces of a resin film, respectively, to form three layers. Here, it preferred that polyethylene film is employed as the resin film, and a double structure fiber of polyethylene/polyester as the nonwoven resin fabrics.

The nonwoven resin fabrics are formed by mixing two types of yarn. The first yarn to mix has a fineness of 7.8dt, while the second yarn has a fineness of 3.3dt. As the dental impression film, it is desirable to use a mixture of the first yarn in 70% - 50% and the second yarn in 30% - 50% as the material for the nonwoven resin fabrics. The dt indicating fineness stands for decitex.

Employed as polyethylene film is, for example, the polyethylene film (brand name "Antibacterial One-Wrap (registered trademark) 220m", heatproof temperature of 110°C) manufactured by Nippon Paper-Pak Co., Ltd. Employed as the double structure fiber of polyethylene/polyester is, for example, a nonwoven fabric (part number "MF-1") manufactured by Shinwa Corp. This material has a feature that heat seal is easy, and its bonding strength is excellent.

Regarding the sizes of the resin film and nonwoven resin fabric, the width of the nonwoven resin fabric is set shorter than the width of the resin film. Then, when the two are overlapped, the nonwoven resin fabric is completely fitted inward of the resin film, thereby preventing poor bonding of impression materials.

### (Manufacturing Process 2)

Next, a virgin pulp is laid under the lowermost nonwoven resin fabric. Then, these are wound around a roll to form a rolled whole.

By laying a virgin pulp in this way, the surface of the nonwoven resin fabric can be protected, at the same time, impurities are prevented from adhering to the nonwoven resin fabric, and the nonwoven resin fabrics are prevented from adhering to each other. By winding around a roll to form a rolled whole, uneven pressure is prevented from being applied between the resin film and the nonwoven resin fabrics. This allows the nonwoven resin fabrics to thermally adhere to the opposite surfaces of the resin film without breaking the three-dimensional fiber structure of the nonwoven fabrics.

When the whole is made into the rolled form, this state can be maintained by using strings, clamps or the like. Regarding the size of the virgin pulp, the width of the virgin pulp is set larger than the width of the resin film. When a long product is employed for each of the resin film, nonwoven resin fabrics and virgin pulp, it is cut to a suitable length after making it into a rolled form.

### (Manufacturing Process 3)

Finally, they are heated at a predetermined temperature with a heating device, thereby causing the nonwoven resin fabrics to thermally adhere to the opposite surfaces of the resin film. Regarding the heating temperature by the heating device, it is preferred to set it to or above the heatproof temperature of the resin film and within a range of 118 to 135°C. Specifically, it is appropriate to set the heating temperature to 118 to 121°C and carry out heating for about five minutes where the polyethylene film (brand name "Antibacterial One-Wrap (registered trademark) 220m", heatproof temperature of 110°C) manufactured by Nippon Paper-Pak Co., Ltd. is employed as polyethylene film, and the nonwoven fabric (part number "MF-1") manufactured by Shinwa Corp. is employed as the double structure fiber of polyethylene/polyester.

Here, as the heating device, it is preferred to use a high-pressure steam sterilizer. This high-pressure steam sterilizer refers to an instrument that carries out saturated vapor sterilization under a pressure exceeding atmospheric pressure. This is an optimal heating device for manufacture of the dental impression film, which causes the nonwoven resin fabrics to thermally adhere to the opposite surfaces of the resin film by setting a predetermined sterilization temperature and sterilization time. The heating device is not limited to this. For example, a handheld heater can be used. This type of heater has two arms which may be used to pinch the polyethylene film and polyethylene/polyester therebetween while being heated, thereby achieving thermal adhesion of the two materials.

When heating what is made in many winds and thickly into a roll form, adjustment may be made to set a long heating time and a somewhat high temperature, and a stand may be used to hold the roll in a floating state, since there is a possibility of causing thermo compression bonding due to its own weight acting on the lower end portion contacting the support surface of the heating device. Further, it is preferable to use a stand that can hold the roll in an upstanding state, in order to avoid an adhesion failure due to vapor becoming water drops in the film.

### [Embodiment 6]

A flow chart of an impression technique (two-step process) using the dental impression film according to this invention (hereinafter called registered trademark "miracle fit") will be described hereinafter as Embodiment 6.

A method of using the dental impression films according to Embodiment 1 to Embodiment 5 this invention includes a step of preparing a wax tray and an impression step for taking impressions. Each of these steps will be described in the order of operations. The method of using the dental impression films will be described taking an example of forming a crown. This crown is, in this prosthesis production, attached to a position between teeth not to be treated.

### <Method of Producing a Wax Tray Prepared Beforehand: Wax Tray Producing Step>

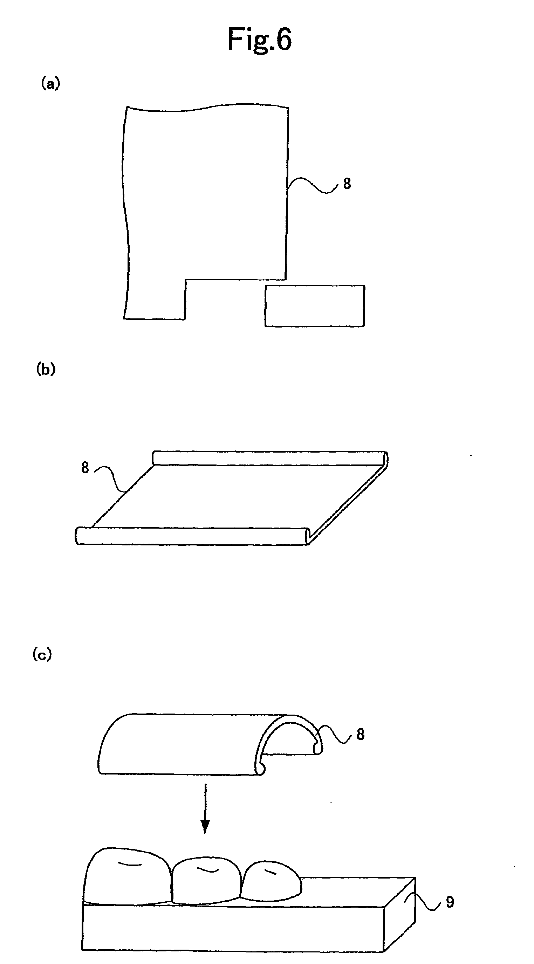

A wax tray is produced through the following substeps (1) - (3):
(1) Cut tool wax 8 (dental paraffin wax) to a size capable of sufficiently covering a formation tooth and contact (contact point between teeth) [see Fig. 6 (a)].
(2) Bend edges of the tool wax 8 inward to secure a thickness and strength for fixing the miracle fit. In this case, the tool wax 8 may be used in two plies or a thick piece may be used. As shown in Fig. 6 (b), the edges bent inward have a stronger force for holding alginate. The wax of an occlusal surface (upper part) should be thick to avoid deformation of the wax tray and mixing-in of bubbles at a time of primary impression.
(3) Press the tool wax 8 on a plaster model 9 with a metal core in order to bend the tool wax 8 to the shape of a U-character [see Fig. 6 (c)]. Then, a fine adjustment of shape is carried out by lightly pressing the bent tool wax 8 in the patient's oral cavity. In this step, instead of the plaster model with a metal core, a commercially available all jaw teeth model made of plastic (silicone) may be used to bend the tool wax 8 at a site of impression to the shape of a U-character, and press it in the patient's oral cavity for production.

At this time, if an attempt is made to press and produce the wax tray in the patient's oral cavity in one operation, the patient will often complain of pain. It is therefore desirable to divide the pressing into two steps. The operation of pressing the paraffin wax (tray) in the oral cavity described in (3) may be carried out by warming and softening the paraffin wax in two plies with a dental gas torch or burner, which will cause little pain on the patient and simplify the production. On the surface of the wax tray surface for which pressure contact is completed, a line is marked with a dental instrument or the like to indicate a tooth reference point. This tooth reference point is a mark used at a time of inserting the wax tray in the patient. Subsequently, the wax tray is cooled with tap water or the like, whereby the wax hardens to complete a firm wax tray. Since the temperature of the locality and the temperature of tap water are variable, chilled water may be used or hard type paraffin wax of high melting point may be used according to circumstances.

The length of this wax tray will be described. Desirably, the length of the wax tray should cover a tray at a time of secondary impression described hereinafter. At the very least, a wax tray for the teeth including the formation tooth and its required contact (contact point) is required. That is, the wax tray is required to have at least a size to protrude from the crown and cover part adjacent teeth adjoining the crown.

### <Flow Chart of Impression Method (Two-Step Process): Impression Step>

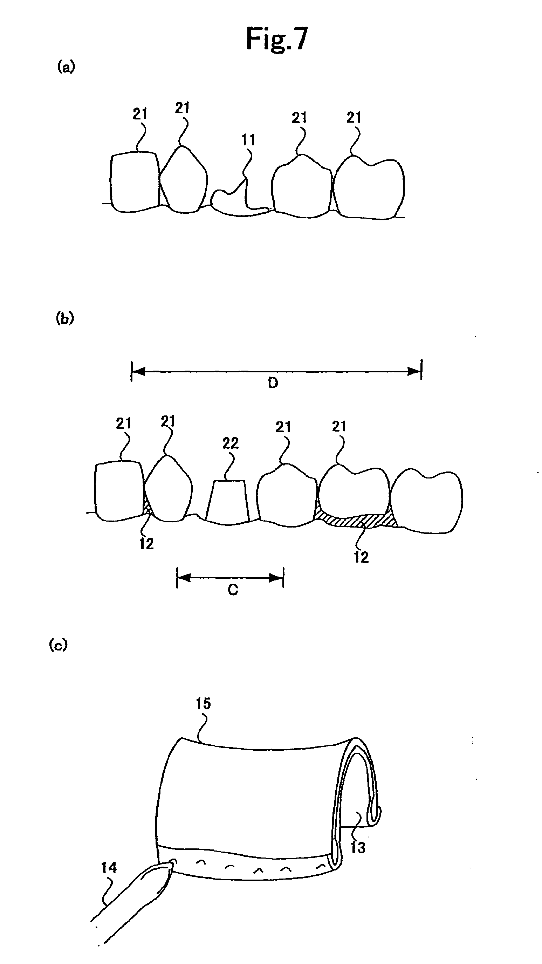

After the production of the above wax tray, impression of the patient's tooth is carried out. This step is executed through the following substeps (4) - (16). It is assumed for this example that, for the reason of one of the patient's teeth being eroded by decay as indicated by sign 11 in Fig. 7 (a), the toothtop substance has been lost, and a crown is to be produced to replace the missing substance. Sign 21 in Fig. 7 (a) denotes teeth not to be treated.
(4) As shown in Fig. 7 (b), the decayed tooth 11 is dug out and removed, and as a substitute an abutment piece is embedded in the position where the tooth 11 existed, to form an anchor tooth 22. This anchor tooth 22 serves as the basis for supporting the crown.
(5) As shown in Fig. 7 (b), the strong undercut and the embrasure of the gingival recession portion of the interdental papilla portion are relieved (buried) with a dental agar impression material 12. And portions where the miracle fit undergoes a strong load when an impression material to be described hereinafter is removed after hardening. In short, this prevents occurrence of a stress that deforms the silicone when demolding the silicone after the silicone impression material permeates to inner parts of the embrasure, and also prevents the silicone from tearing off.
(6) The miracle fit 13 is fitted to the inside of wax tray 15, and is trimmed with scissors, for example, leaving margins of about 3-5mm at edges. It is better if the miracle fit 13 does not protrude from mesiodistal parts (side edges) of the wax tray at this time. When the silicone of the occlusal surface protruding from the tray becomes thin at a time of primary impression, the thin silicone will be lifted at a time of secondary impression or formation of a gypsum mold to be described hereinafter, thereby impairing accuracy at that portion. Therefore, the thinly protruding portions of the silicone are removed before the silicone hardens. However, if the protruding portions of the silicone are thick, accuracy will not be impaired to a serious degree. The length of this wax tray 15 is set to an extent of covering at least part of adjacent teeth 21 at opposite sides of the anchor tooth 22. That is, the length of range C in Fig. 7 (b) corresponds to the length of wax tray 15 at this time. If a more faithful denture mold is desired, it is necessary to lengthen the wax tray 15. The length of wax tray 15 at this time corresponds to the length of range D in Fig. 7 (b), for example. In this case, as described in (5) above, it is necessary to apply the dental agar impression material 12 to the embrasure of the treated tooth 21 beforehand as relief. Otherwise, the silicone impression material will permeate to inner parts of the embrasure.
(7) Poke the edges of the wax at several locations on the cheek lingual side (cheek palate side) from above the miracle fit 13 with an Evans carver 14, to fix the miracle fit 13 to the wax tray 15. [see Fig. 7 (c)].

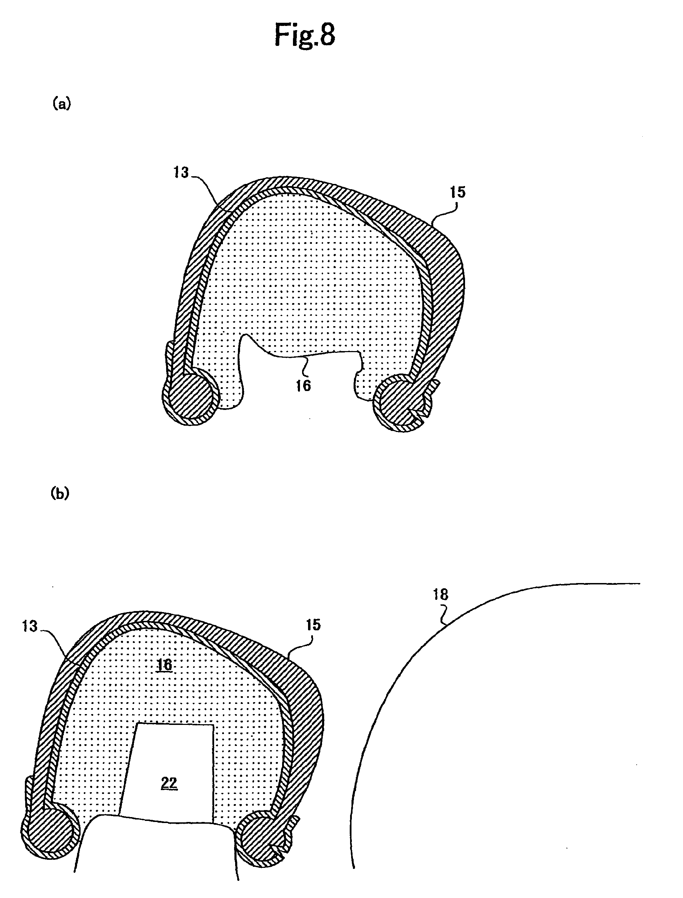

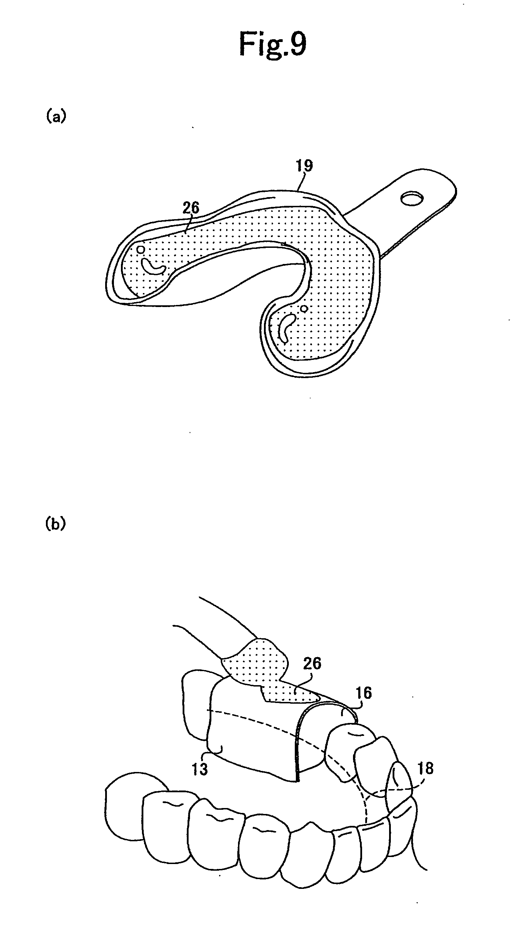

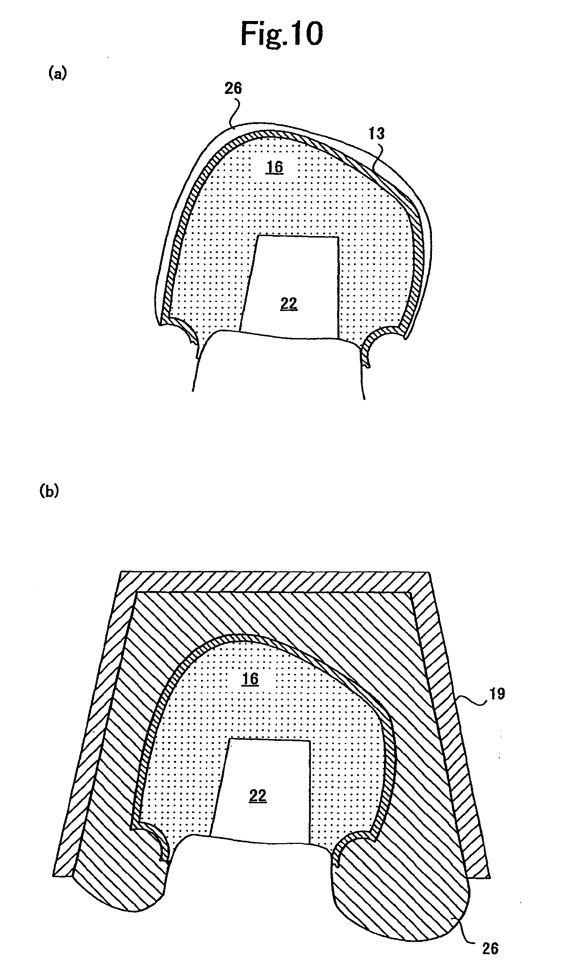

Once the wax tray 15 with the miracle fit 13 fixed thereto is made, the operation enters on impression.
(8) As silicone, the injection type and, according to the case, the light body or normal body type are prepared (impression materials are used in a combination recommended by the makers in order to match the operate time of each silicone and time of retention in the oral cavity, and proper impression materials are selected having regard to the number of formation teeth for which impressions are taken, a flow (fluidity), operate time and time of retention in the oral cavity according to the case).
(9) The operator and an assistant start malaxation of the silicone at the same time.
(10) The silicone of the injection type malaxated by the operator is put into a syringe, and the silicone is injected from the syringe toward impression margins (minute portions). Subsequently, the remaining injection silicone is applied to the anchor tooth 22 (and adjacent teeth) and other occlusal surface portions (a special syringe is convenient).
(11) The assistant malaxates light body or normal body type silicone 16 and, as shown in Fig. 8 (a), spreads it over the inner surface of the miracle fit 13 of the wax tray produced in (3) above.
(12) The operator finishes using the injection silicone, at the same time receives from the assistant the wax tray 15 holding the silicone, and presses the wax tray so that a line mark and a tooth reference point may coincide [see Fig. 8 (b)]. The line mark is a mark put on the wax tray. The tooth reference point is a side of another tooth 21, for example. Part of the silicone 16 on the tooth (certainly portions concerned in occlusion) protruding thinly from the wax tray is removed. In Fig. 8 (b), sign 22 is the anchor tooth, and sign 18 is the tongue.
(13) The alginate 26 is malaxated in a standard powder/liquid ratio specified by the maker according to the hardening characteristic of the silicone 16, and is placed on a metal tray 19 having undergone a trial test [see Fig. 9 (a)]. Time is measured from the time of wax tray pressure contact, and malaxation time is adjusted so that placement of the alginate may be completed at elapse of the time of retention in the oral cavity of the silicone 16.
(14) Check that the silicone 16 in the oral cavity has hardened, take only the wax tray 15 out of the oral cavity [see Fig. 9 (b)], and expose the miracle fit 13. Take, with a finger, a small amount of the alginate 26 which has been placed on the metal tray, and rub the alginate 26 into the miracle fit 13 on the hardened silicone surface with the finger. At this time, a section in the oral cavity is as shown in Fig. 10 (a).
(15) Deposit the metal tray 19 from top, and press it on the patient's tooth [see Fig. 10 (b)].
(16) The metal tray 19 is taken out of the oral cavity after the alginate 26 hardens, thereby taking en bloc out of the oral cavity, the miracle fit 13 buried in the alginate 26, and the silicone 16 which is now a precise mold of the defective part of the patient's tooth.

Bite taking can be carried out using the wax tray detached. Gypsum is poured into the silicone alginate impression material in accordance with a common method. The crown is produced based on the mold of gypsum obtained. Since accuracy near a silicone impression is taken, a temporary crown may be attached in order to prevent tooth movement until completion and attachment of a prosthesis, which will enable attachment of the prosthesis in a short time.

### [Embodiment 7]

Next, Embodiment 7 relating to production of a plate denture will be described. A plate denture is different from what is called a crown (covering) like the crown in Embodiment 6, and is a set of false teeth or a partial denture with artificial gums. When producing such a plate denture also, the dental impression film in Embodiment 1, Embodiment 3 or Embodiment 4 according to this invention can be used. Embodiment 7 assumes a patient who has had a plate denture produced. The construction of Embodiment 7 is effective where, even if a plate denture is once produced, a new plate denture needs to be produced after the gums become thin, for example. This embodiment is very significant in that a plate denture needs to be replaced with a new one in several years.

The plate denture according to Embodiment 7 is produced by performing an adhesive liquid applying step S1, a film attaching step S2, an adhesive applying step S3, a silicone applying step S4, a mucosal surface impression step S5 and a tooth surface impression step S6. Details of these steps will be described in order.

### <Adhesive Liquid Applying Step S1>

First, an adhesive liquid is applied to the dental impression film in Embodiment 1. The adhesive liquid is applied to one surface of the polyethylene polyester films 20 shown in Fig. 1.
Then, the surface to which the adhesive liquid is applied becomes adhesive. This surface will be called the bonding surface. Since the dental impression film in Embodiment 1 has the opposite surfaces formed of the same material, the surface to which the adhesive liquid is applied is not limited to a particular surface. However, the adhesive liquid will be applied to the untreated surface of the adhesive film 50 when the dental impression film of Embodiment 3 is used, and to the untreated surface of the silicone impression material film 70 when the dental impression film of Embodiment 4 is used.

### <Film Attaching Step S2>

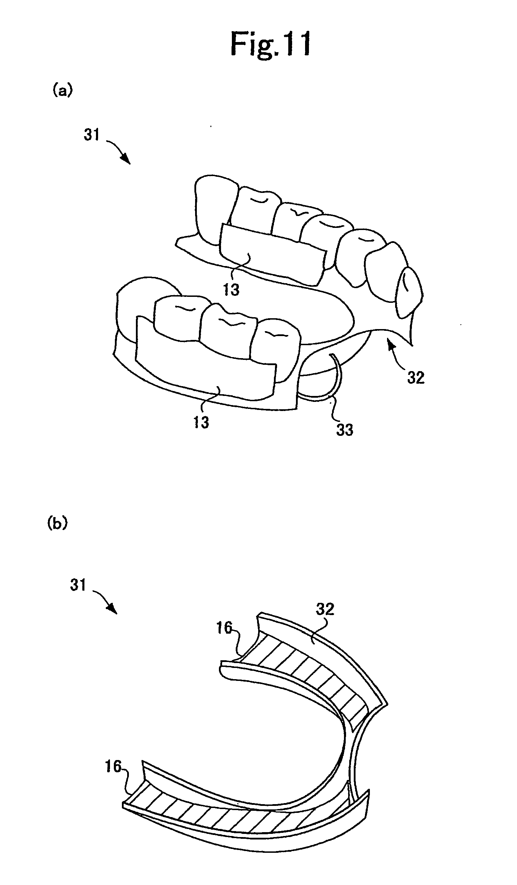

And, as shown in Fig. 11 (a), miracle fits 13 having the above adhesive property are applied one after another to portions of teeth and plate except the occlusal surfaces such as of the crowns of grinding teeth of the original plate denture 31. At this time, the miracle fits 13 are not applied to the entire plate, but portions of the plate that contact the patient's gums and their peripheral portions are excepted. In this way, adhesion of the plate denture to the alginate 26 becomes good. In addition, separation of the plate denture from the alginate 26 also becomes good. Exposed surfaces of miracle fits 13 serve as permeation surfaces permeated by the alginate 26. The original plate denture is one example of solids of this invention.

When the shape of the original plate denture is widely different from the shape of the patient's gums, it is preferable to apply a mucosa regulator to contact surfaces of the gums of the plate denture, as a measure of accommodating the shape of the inner surface of the original plate denture to the shape of the patient's gums to some extent.

### <Adhesive Applying Step S3 and Silicone Applying Step S4>

Next, an acrylic adhesive is applied to contact portions 32 and their peripheries of the gums of the original plate denture 31. The meaning of applying this adhesive will be described hereinafter. After completion of the adhesive application, the silicone 16 is coated thereon. In this way, the contact portions 32 of the gums in the form of a U-shaped recess is filled up with the silicone 16 as shown in Fig. 11 (b).

### <Mucosal Surface Impression Step S5>

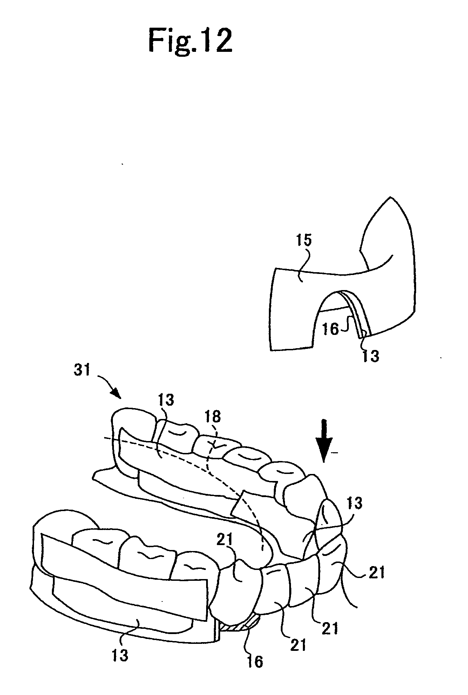

At this time, the original plate denture 31 is fitted in the patient's oral cavity. Then, as shown in Fig. 12, the patient's gums and silicone 16 contact, and the silicone will soon harden. In this way, a mold of the patient's gums will be impressed on the inside of the original plate denture 31. Sign 21 in Fig. 12 represents the patient's remaining teeth.

After fitting the original plate denture 31 in the patient's oral cavity, the patient is asked to make tongue movement and jaw movement within the operate time of silicone 16. Once the retention time of silicone 16 starts, and before the silicone hardens, the patient is asked to keep still with the upper and lower jaws engaged. In this way, a dynamic impression (also called a functional impression) of the patient's gums can be taken, thereby providing a plate denture more suitable for use.

### <Tooth Surface Impression Step S6>

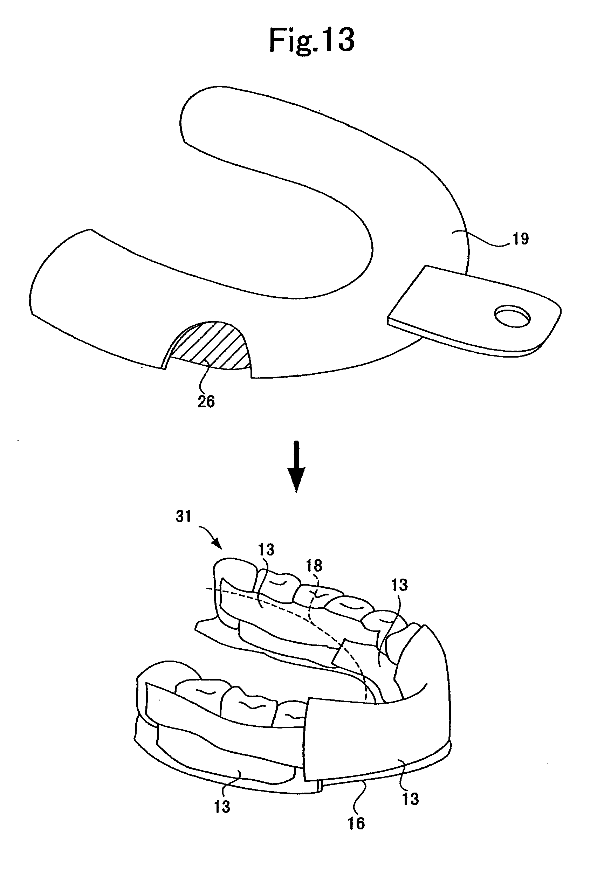

Thereafter, as shown in Fig. 12, the wax tray 15 is fitted in the patient's oral cavity so as to cover all the remaining teeth 21. The subsequent operations are carried out as described in Embodiment 6. That is, the operations of (8) - (16) in Embodiment 6 are carried out. Fig. 12 shows up to the step of putting the wax tray 15 on the remaining teeth 21. Although the wax tray 15 appears to have a cutout, this is provided for the purpose of illustrating the interior of wax tray 15. In practice, no such cutout exists. The length of this wax tray 15 is set to an extent of covering all the remaining teeth 21 pinched by the resin teeth reproduced on the plate denture 31. Therefore, the wax tray 15 has a construction to protrude from the end of the remaining teeth 21 adjoining the plate denture. Fig. 13 shows up to the step of pressing on the patient's teeth the metal tray 19 filled with the alginate 26. Although the metal tray 19 appears to have a cutout exposing the alginate 26, this is provided for the purpose of illustrating the interior of metal tray 19. In practice, no such cutout exists.

In Embodiment 7, how a step corresponding to (16) in Embodiment 6 is executed will be described. In Embodiment 7, the hardened alginate 26 placed in the metal tray 19 is taken out of the patient's oral cavity in a state of the original plate denture 31 buried therein. The hardened alginate 26 has, exposed on the impression surface thereof, the silicone 16 covering the surfaces of the contact portions 32 of the original plate denture 31, and the silicone 16 covering the surfaces of the miracle fits 13. A mold of the patient's gums is accurately transferred to the silicone 16 covering the surfaces of the contact portions 32, while a mold of the patient's remaining teeth 21 is accurately transferred to the silicone 16 covering the surfaces of the miracle fits 13. Based on this, a new plate denture can be prepared. This preparation method can use an existing method using gypsum.

Since the alginate 26 covers the entire jaw, portions of the plate denture not covered by the miracle fits 13, i.e. the occlusal portions of the plate denture, have also been transferred. Since the silicone 16 is not applied to such portions at all, when the metal tray 19 is pressed, the occlusal portions of the plate denture directly contact the alginate 26, and the occlusal portions of the plate denture are transferred onto the alginate 26. Such occlusal portions of the plate denture contacting the alginate 26 are necessary for reproducing an occlusion situation of false teeth. In order to inhibit wobbling in the oral cavity, the plate denture has a clasp 33 for fastening to a remaining tooth 21 [see Fig. 11 (a)]. The tooth to which the clasp 33 is fastened is called a hinge tooth. Since a mold of this hinge tooth is taken with the silicone 16, this clasp 33 can be produced precisely. The device for fastening the plate denture to the remaining tooth is not limited to this clasp 33. The construction of Embodiment 7 can be adapted to other devices than the clasp 33.

According to the construction of Embodiment 7, as described above, the patient's gums and teeth can be transferred faithfully while saving on the expensive silicone 16. That is, the patient's plate denture is used in transferring the patient's gums. Since the shape of the plate denture is produced according to the patient, it is almost an imitation of the shape of the patient's gums. This plate denture may be regarded as a tray (support for the impression material), and the silicone 16 may be placed therein. Then, the amount of silicone 16 used can be reduced to a minimum.

When removing the metal tray 19 out of the oral cavity, it may be taken out of the oral cavity in a state of supporting the plate of the plate denture. Then, excessive stress is not applied to the miracle fits 13, to realize a more faithful denture mold.

In the film attaching step S2, the miracle fits 13 are applied to the original plate denture. This allows the original plate denture to be collected. That is, the original plate denture is buried in the hardened alginate 26. However, the miracle fits 13 are arranged in positions between the original plate denture and hardened alginate 26. Therefore, the original plate denture and hardened alginate 26 do not adhere to each other. Since the miracle fits 13 can be stripped from the original plate denture simply, the original plate denture can be collected easily even after carrying out Embodiment 7.

This invention is not limited to the foregoing construction, but may be modified as follows:
(1) Each embodiment described above is adapted for medical use. However, this invention can be adapted for general extractive industry having mold manufacturing steps.
(2) In the embodiments described above, Embodiment 6 has been made an example of crown manufacture. However, the technique of Embodiment 6 is not limited to crown manufacture. Embodiment 6 is applicable to manufacture of plate dentures instead of crowns.
(3) Embodiment 1 described above may be implemented in a modified form. Specifically, metal foil can be used instead of polyethylene film 10, and metal nets instead of polyethylene/polyester films 20. With this construction, the dental impression film will have a certain shape maintaining capability. Therefore, the wax tray 15 described in Embodiment 6 is not required, and substeps (1) - (3) in Embodiment 6 can be omitted. The metal foil and metal nets are electrically welded in grid form with a spacing of about 2mm in between. A construction may be provided in which the polyethylene/polyester films 20 are attached to the opposite surfaces of metal foil by thermal adhesion or an acrylic adhesive. Aluminum may be suitable as the material for metal foil and metal nets, but this may be changed to copper. In this case, copper and a polysulfide rubber impression material become chemically bonded, and this can dispense with the metal net disposed on one surface of the metal foil. As in Embodiment 2, the dental impression film may include two layers. Specifically, a combined impression of the polysulfide rubber impression material and alginate impression material is possible. In this case also, as noted above, the polyethylene/polyester film 20 may of course be used instead of the metal net.
   In the construction having the metal net disposed on one surface of metal foil, selecting an adhesive applied to the metal foil allows the metal foil to be a material other than copper. In this case, as the adhesive, an adhesive specified by the impression material maker (silicon tray adhesive, rubber adhesive) is selected. These adhesives are capable of adhering to metallic foil. (4) Instead of polyethylene film 10 in each embodiment described above, a vinyl chloride film may be used.

### [INDUSTRIAL UTILITY]

As described above, the dental impression film and the method of manufacturing the dental impression film according to this invention are suitable for the medical field.

## Claims

1. A dental impression film for use in a combined impression which overlaps two types of dental impression materials different in quality, wherein the two types of dental impression materials different in quality are made joinable by laminating a layer having bonding capability, or attaching projections having bonding capability, between the dental impression materials having hardened, on opposite surfaces of the dental impression film at a time of impression taking.

2. The dental impression film according to claim 1, wherein the dental impression materials comprise a combination of a silicone impression material and an alginate impression material.

3. The dental impression film according to claim 1 or 2, wherein the dental impression film comprises films of polyethylene and polyester having bonding capability between silicone impression material and alginate impression material, laminated by thermal adhesion on opposite surfaces of a polyethylene film.

4. The dental impression film according to claim 1 or 2, wherein the dental impression film comprises a layer of silicone impression material fixed to one surface of a film having bonding capability between silicone impression material and alginate impression material.

5. The dental impression film according to claim 1 or 2, wherein the dental impression film comprises looped projections, which will become rigidly and inseparably entwined when permeated by and hardened with the alginate impression material, attached to a surface, to be bonded to the alginate impression material, of the film having bonding capability with respect to the silicone impression material.

6. A method of manufacturing a dental impression film comprising laying nonwoven resin fabrics on opposite surfaces of a resin film, respectively, to form three layers, laying a virgin pulp under a lowermost nonwoven resin fabric, then winding these around a roll to form a rolled whole, and subsequently heating at a predetermined temperature with a heating device, thereby causing the nonwoven resin fabrics to thermally adhere to the opposite surfaces of the resin film.

7. The method of manufacturing a dental impression film according to claim 6, wherein the resin film is a polyethylene film, and the nonwoven resin fabrics are a double structure fiber of polyethylene/polyester.

8. The method of manufacturing a dental impression film according to claim 6 or 7, wherein the heating temperature by the heating device is set to or above a heatproof temperature of the resin film and within a range of 118 to 135°C.

9. The method of manufacturing a dental impression film according to any one of claims 6 to 8, wherein the heating device is a high-pressure steam sterilizer.

10. A dental impression film for use in a combined impression for joining a dental impression material to a solid, wherein the dental impression film has a permeation surface permeable by the dental impression material and an attachment surface for attaching to the solid, the permeation surface having, laminated thereon, a layer having bonding capability, or attached thereto, projections having bonding capability with respect to the dental impression material having hardened, the attachment surface being coated with an adhesive material to be attachable to the solid, thereby being capable of joining the dental impression material to the solid.
